(19) Europäisches Patentamt

European Patent Office

Office européen des brevets

(11) Publication number: 0 380 607 B1

# EUROPEAN PATENT SPECIFICATION

(45) Date of publication of patent specification: 14.12.94

(21) Application number: 89906908.2

(22) Date of filing: 17.05.89

(86) International application number:
PCT/US89/02073

(87) International publication number:
WO 89/11296 (30.11.89 89/28)

The file contains technical information submitted
after the application was filed and not included in
this specification

(51) Int. Cl.⁵: **A61K 39/395**, C07K 15/16,
C12N 15/00, G01N 33/577,
G01N 33/543

(54) ANTI-IDIOTYPE ANTIBODIES TO ANTI-HUMAN HIGH MOLECULAR WEIGHT-MELANOMA ASSOCIATED ANTIGEN.

(30) Priority: 17.05.88 US 195577

(43) Date of publication of application:
08.08.90 Bulletin 90/32

(45) Publication of the grant of the patent:
14.12.94 Bulletin 94/50

(84) Designated Contracting States:
AT BE CH DE FR GB IT LI LU NL SE

(56) References cited:
US-A- 4 536 479
US-A- 4 699 880
US-A- 4 731 237

JOURNAL OF IMMUNOLOGY, vol. 129, no. 6,
1982; pp. 2734-2738&NUM;

EX. CELL RESEARCH, vol. 137, 1982; pp.
89-94&NUM;

(73) Proprietor: FERRONE, Soldano
51 Old Orchard Lane
Scarsdale, NY 10583 (US)

Proprietor: RAYCHAUDHURI, Syamal
3716 Carmel View
San Diego, CA 92130 (US)

(72) Inventor: Ferrone, Soldano
51 Old Orchard Lne
Scarsdale, NY 10583 (US)

(74) Representative: Goldin, Douglas Michael et al
J.A. KEMP & CO.
14, South Square
Gray's Inn
London WC1R 5LX (GB)

NATURE, vol. 308, 1984; p. 457&NUM;

CANCER RESEARCH, vol. 46, October 1986, US; KANTOR et al., pp. 5223-5228&NUM;

JOURNAL OF BIOLOGICAL CHEMISTRY, vol. 259, no. 20, 25 October 1984, US; BUMOL et al., pp. 12733-12741&NUM;

CANCER RESEARCH, vol. 47, no. 16, 15 August 1987, Philadelphia, PA (US); M. KUSAMA et al., pp. 4312-4317&NUM;

JOURNAL OF IMMUNOLOGICAL METHODS, vol. 95, 1986, Elsevier Science Publ. B.V., Amsterdam (NL); M. TSUJISAKI et al., pp. 47-55&NUM;

R. GALLO et al., "Monoclonals and DNA Probes in Diagnostic and Preventive Medicine", 1987, Raven Press, New York, NY (US); pp. 101-110&NUM;

PROCEEDINGS OF THE AMERICAN ASSOCIATION OF CANCER RESEARCH, vol. 28, March 1987; A. MITTELMAN et al., p. 390, no. 1548&NUM;

## Description

### Field of the Invention

This invention relates to the development of antibodies which are specifically designed to enhance immunological activity by the host against a tumor. More particularly, investigators have treated patients suffering from melanoma with antibodies which are directed to antigens contained on the melanoma cells. The High Molecular Weight - Melanoma Associated Antigen (hereinafter "HMW-MAA") is an appropriate target for such immunotherapeutic approaches to melanoma. 225.28 is a monoclonal antibody which recognizes an epitope of undefined structure on the HMW-MAA molecule. This invention is in the field of anti-idiotypic antibodies to anti-HMW-MAA antibody 225.28 and its immunological equivalents. The anti-idiotype antibodies MF11-30 and IMelpg1 to antibody 225.28 have been discovered and evaluated in animal model systems. MF11-30 also has been evaluated in patients with malignant melanoma. Diagnosis, monitoring and treatment of tumors, such as melanoma or other diseased cells which express the HMW-MAA epitope recognized by antibody 225.28, may be carried out by the administration and application of such anti-idiotypic antibodies.

### Background of the Invention

#### Introduction

Antigens are substances which can induce an immune response. One typical immune response is antibody production. Antibodies are produced by B-cell lymphocytes. Another typical response is the stimulation of T-cells. (T-cells are another type of lymphocyte which have undergone differentiation in the thymus gland). T-cells are capable of destroying abnormal cells such as those infected by certain types of viruses or other pathogens. T-cells also are involved in antibody production by B-cells. Accordingly, an antigen can react either with antibodies or with receptors on the T-cells which it stimulates. Antibodies and T-cell receptors are specific for the inducing antigen.

Antibodies and T-cell receptor molecules possess variable regions which are responsible for specific antigenic recognition. The region of the antigen that actually binds to the antibody or T-cell receptor is termed the antigenic determinant or "epitope." The variable regions of antibodies and T-cell receptors contain determinants, or "idiotypes" which also are immunogenic and initiate anti-idiotype ("anti-id") immune responses.

More particularly, idiotopes are associated with the variable regions of antibody and T-cell receptors. These variable regions confer antibody and T-cell receptor specificity for antigens. Idiotypes are immune system markers on antibodies and T-cell receptors. An idiotype is immunologically defined by reactivity with more than one anti-idiotypic antibody which recognizes an idiotopic determinant or idiotope within a given idiotype; i.e., an idiotype is made up of a collection of idiotopes. Idiotopes are best defined by their binding to monoclonal anti-idiotypic antibodies.

It also should be noted that idiotopes are distinct from isotypic (immunoglobulin class-specific), xenotypic (species specific) and allotypic (certain population subgroup specific) determinants.

Each antibody and T-cell receptor has at least one paratope which is the binding site for an antigen determinant (the epitope). A paratope may serve as an idiotope, i.e., the paratope may stimulate an anti-idiotypic response in which, like the original antigen, the anti-idiotypic antibodies bind to the paratope. A subset of anti-paratope anti-idiotope antibodies may mimic the immunologic properties of the original antigen. In addition to the anti-paratopic anti-ids which mimic the original antigen, other anti-id antibodies define antibody and T-cell receptor idiotopes which also participate in the regulation of immune responses. These idiotypes are termed regulatory idiotypes and they are not necessarily "internal images" of the original antigen. For a general discussion of these background principles, see Burdette, S. and Schwartz, R., New Eng. J. of Med. 317:219 (1987).

#### The Idiotypic Network

In any given species or individual, the number of different antibodies and T-cell receptors is very large ($>10^7$). Each antibody and T-cell receptor expresses several idiotopes which are linked through a regulatory network to complementary anti-idiotopes. Jerne, N., Ann. Immunol. 1256:373 (1974), proposed that the immune system is regulated by a network composed of idiotypes and anti-idiotypes. There is a large amount of experimental evidence indicating that the function of the idiotypic network is to maintain the

3

homeostasis of the immune system and to regulate immune responses. As idiotypes are expressed by B-cells and T-cells, the idiotypic network encompasses both humoral (antibody) and cellular (T-cell mediated) immune responses. Specific immune responses are regulated by specific anti-idiotypic antibodies either on a one to one basis, or in groups through shared idiotopes. These anti-idiotypic antibodies are termed "regulatory anti-idiotypes" and they do not necessary represent the internal image of the target antigen. See, Goldberg, B., et al., J. Exp. Med. 158:515 (1983).

Anti-idiotype Therapy

Recently, the use of "internal image" anti-idiotypes in therapy has been suggested and demonstrated in several experimental systems (EPO App. No. 848105169, filed October 15, 1984, Pub. No. 0141783; Nisonoff, A., and Lamoyi E., Clin. Immunol. Immunopathol. 21:397 (1981); Kennedy, R.C., et al., Biotechniques 3:4040 91985)). These internal image anti-idiotypes have been used as surrogates for antigens in generating specific immune responses against viral, bacterial and parasitic infections and cancers (Herlyn, D., et al., Science 232:100 (1986); Raychaudhuri, S., et al., J. Immunol. 137:1743 (1986).

Currently, internal image polyclonal and monoclonal anti-idiotypes are being used for active immunotherapy in animals and man. The process of generating such internal image anti-idiotypes is as follows: 1) an antibody is made against a tumor antigen or an infectious agent. This antibody is called "Ab1"; 2) anti-idiotypic antibodies ("Ab2s") are made against the first antibodies. These anti-ids (Ab2s) are screened for the expression of idiotypes which mimic the immunological properties of the initial antigen (such as that expressed by a tumor or infectious agent). This screening consists of inhibiting the Ab1-Ab2 binding by the original antigen and the biological testing of the anti-idiotypes (Ab2) as a surrogate antigen in vivo. This testing is done in animals and has been demonstrated previously for internal image anti-idiotypes representing tumor antigens and infectious agents (Kennedy, R.C., et al., Biotechniques 3:4040 (1985); Herlyn, D., et al., Science 232:100 (1986); Raychaudhuri, S., et al., J. Immunol. 137:1743 (1986).

Prior art efforts to generate useful active tumor immunotherapies, however, have met with unpredictable success. One may postulate several reasons for the limited success and unpredictable results seen in the prior art.

First, many tumor antigens are difficult to isolate. Tumor antigens often are not present in significant concentration in tumor masses or on individual cultured tumor cells. Secondly, tumor antigens often are poorly immunogenic. The poor immunogenicity may be a result of the presence of other constituents of tumor cells which suppress immunologic reactivity, or because the antigens are not uniquely expressed by tumor cells. Generally, antigens which the immune system recognizes as "self" will not provoke a significant immune response. One prior art approach to tumor immunotherapy was to use internal image anti-idiotype antibodies which can be manufactured in large quantities and may be more immunogenic than natural antigens.

However, prior art efforts to generate useful internal image anti-idiotypes also have met with unpredictable success. With reference to the idiotypic network, many anti-idiotype antibodies, for reasons that are unclear, simply are unable to induce efficacious immune responses in vivo despite analyses indicating that they mimic the properties of the original antigen. Even anti-idiotype antibodies which demonstrate the proper characteristics in vitro, or even partially in vivo, oftentimes are unable to stimulate the immune network to generate a therapeutically effective immune response. Presumably, these ineffective internal image Ab2s do not induce the appropriate regulatory circuits of the immune network of idiotype/anti-idiotype interactions to elicit effective immunity. Against this backdrop of unpredictability and uncertainty, the inventive anti-idiotype antibodies herein described have been successfully tested in patients with advanced melanoma.

One appropriate target to develop immunotherapeutic approaches to melanoma is the high molecular weight melanoma-associated antigen (HMW-MAA) (also referred to as the melanoma-associated chondroitin sulfate proteoglycan antigen ("MPG"). Our results show that attempts to induce cellular and humoral responses against HMW-MAA may result in intervention of tumor growth. The HMW-MAA consists of two noncovalently associated glycopolypeptides. One has an apparent molecular weight of 280K, and the other has an apparent molecular weight greater than 440K. HMW-MAA is synthesized and expressed by human melanoma cells (Spiro, R.C. et al. J. Biol. Chem. 264:1779 (1989); Esko, J.D., et al., Science 241:1092 (1988). Proteoglycans are glycoproteins with glycosaminoglycan (GAG) polysaccharide chains covalently attached to the serine residue in their core. The HMW-MAA core protein is initially translated as a precursor with a molecular mass of 240K with asparagine-N-linked oligosaccharides of the high mannose type. Trimming and subsequent processing of the N-linked oligosaccharide to the "complex" form results in the conversion of the 240K component to a 250K form. The addition of chondroitin sulfate GAG side chains to the 250K core protein converts it to a high molecular weight (>450K) proteoglycan form. Both 250K and >450K forms are expressed on the melanoma cell surface (Spiro, R.C. et al., J. Biol. Chem. 264:1779 (1989). MPG plays a key role in determining the fate of tumor growth and metastasis (Herlyn, M. et al., Ann. Rev. Immunol. 6:283 (1988); Robertson, N.P. et al., Cancer Res. 49:1816 (1989). Cell surface protein molecules are involved in interactions with adjacent cells and form complex footpads that make contact with substratum (Herlyn, M., et al., Ann. Rev. Immunol. 6:283 (1988). Antibodies to HMW-MAA block chemotactic and chemokinetic motility of cells (Herlyn, M., et al., Ann. Rev. Immunol. 6:283 (1988), de Vries, J.E., et al., Int. J. Cancer 38:465 (1986)). The anti-HMW-MAA antibodies cause the reduction of melanoma colony formation in soft agar (Harper, J.R., J. Natl. Cancer Inst. 71:259 (1983)).

Mittelman et al (Proc. Am. Assoc. Cancer Res. Vol. 28, 1987 p. 390, Abstract 1548) reports that murine anti-idiotypic monoclonal antibodies (MoAb) to anti HMW-MAA MoAb's 149.53, 225.28 and 763.74 were developed and characterised in their specificity. The anti-idiotypic MoAb MF9-36 to MoAb 149.53 and the anti-idiotypic MoAb MF11-30 to MoAb 225.28 were tested in a Phase I clinical trial.

The applicants have identified two anti-idiotype antibodies, MF11-30 and IMelpg1 and their equivalents, which are capable of reacting with (1) the idiotope of the murine monoclonal antibody 225.28 and derivatives thereof or their immunological equivalents; or (2) any monoclonal antibody secreted by hybridomas from any species having the same immunological characteristics as 225.28; or (3) any polyclonal antisera from any species having the same immunological characteristics as 225.28, which is reactive with a specific determinant (epitope) of a high molecular weight melanoma associated antigen (HMW-MAA). The anti-idiotype antibodies MF11-30 and IMelpg1 were made against 225.28 and have been shown to inhibit the binding of 225.28 to the human melanoma cell line Colo 38, as well as to other cell lines (data not shown). We have found that MF11-30 and IMelpg1 can induce relevant anti-tumor responses.

These anti-idiotype antibodies may be used in active and passive immunotherapy, including the diagnosis, monitoring and treatment of melanoma and other disease states associated with the epitope recognised by the 225.28 antibody.

For example, we have successfully used anti-idiotypic antibodies for the active immunotherapeutic induction of an anti-tumor response in tumor patients. Presented herein are the results of studies using anti-idiotypic antibodies (Ab2s) as immunoactivators to potentiate tumor-specific B and T cell responses in melanoma patients.

The present invention is directed to the anti-idiotypic antibody IMelpg1 obtained from the hybridoma deposited with the ATCC under accession no. HB 10134 and antibodies competing with said antibody IMelpg1 to bind to the murine monoclonal antibody 225.28 obtained from the hybridoma deposited with the ATCC under accession no. HB 10141, said antibodies and said antibody IMelpg1 being free of mycoplasma contamination. Any other antibody mentioned within the present application is not part of the invention.

Description of the Drawings

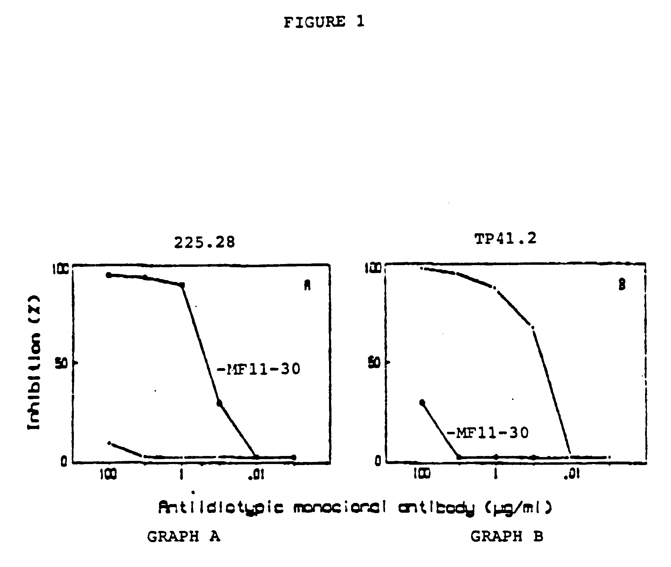

Figure 1 consists of two graphs. Inhibition by anti-idiotypic monoclonal antibody MF11-30 of the binding of $^{125}$I-labeled anti HMW-MAA monoclonal antibody 225.28 to cultured Colo 38 melanoma cells is depicted in Graph A. Graph B depicts the absence of significant inhibition by MF11-30 of the binding of a different 125I labeled anti-HMW-MAA monoclonal antibody (TP41.2) to Colo 38 melanoma cells indicating the specificity of MF11-30 for the 225.28 antibody. The graphs show percent inhibition as a factor of concentration of anti-idiotypic monoclonal antibody in μg/ml.

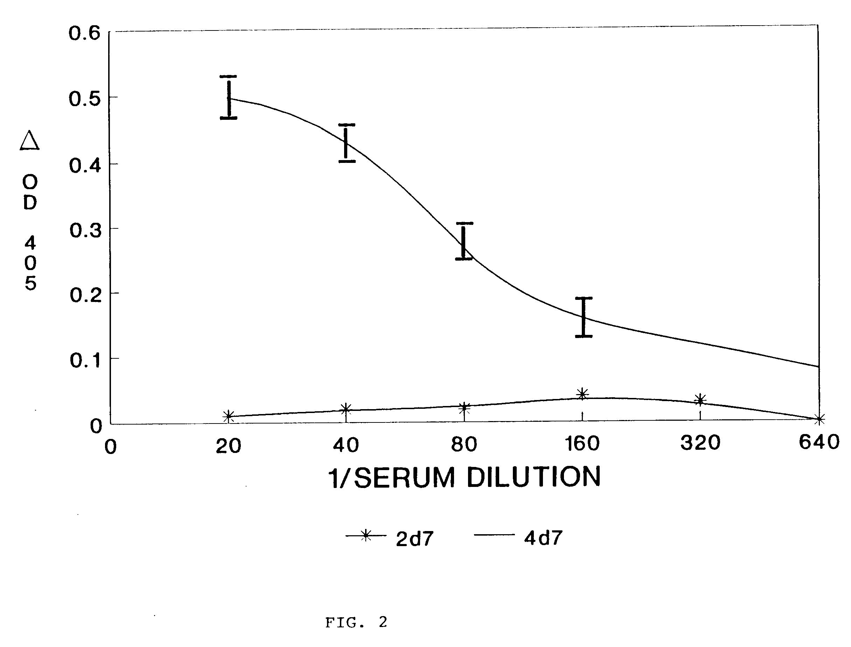

Figure 2 depicts induction of anti-melanoma antibodies by IMelpg1 upon repeated immunizations. Sera collected from rabbits immunized with IMelpg1 or ICO1 were tested 7 days after the second and fourth boost were tested for binding to Colo 38 cells by an ELISA as described in the Materials and Methods section of Part II. The symbols corresponding to different sera are indicated in the figure. Immunization with isotype-matched ICO1 did not induce any detectable anti-melanoma humoral response.

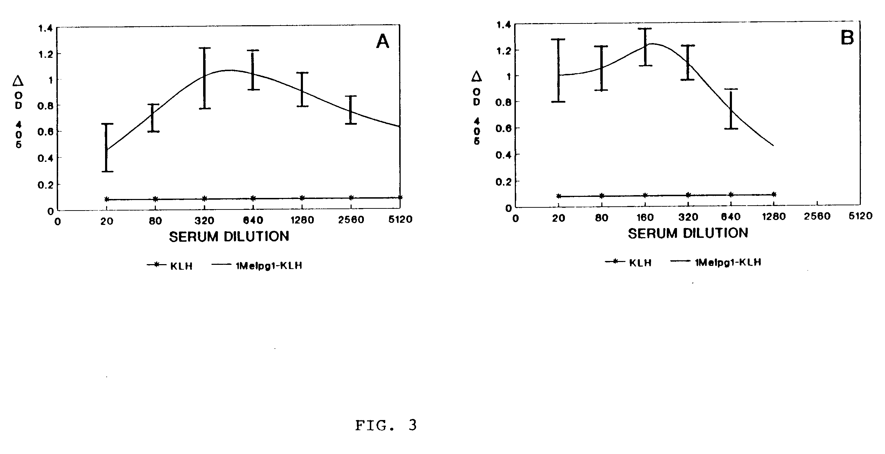

Figure 3 depicts the anti-melanoma antibody response induced by KLH-conjugated IMelpg1. Rabbit sera obtained by immunizing with IMelpg1, IMelpg1-KLH or KLH alone were assayed on Colo 38 cells before (Fig. 3a) and after (Fig. 3b) adsorbing to 3T3 cells by an ELISA as described in the Materials and Methods section of Part II. Again, the symbols corresponding to different sera are indicated in the figure.

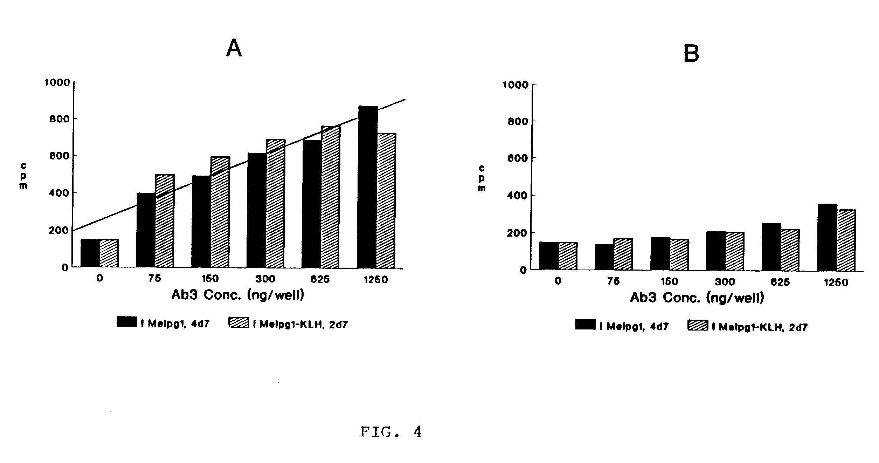

Figure 4 depicts the binding of affinity purified Ab3 to Colo 38 cells. Rabbit sera obtained by immunizing with IMelpg1-KLH were affinity purified and tested for binding to Colo 38 cells (Fig. 4a) or to KLH (Fig. 4b) by a radioimmunoassay as described in the Materials and Methods section of Part II. Representative results obtained with two rabbits are shown. Twenty nanograms of purified 225.28 gives 1200 cpm in a direct binding assay on a Colo 38 cell plate.

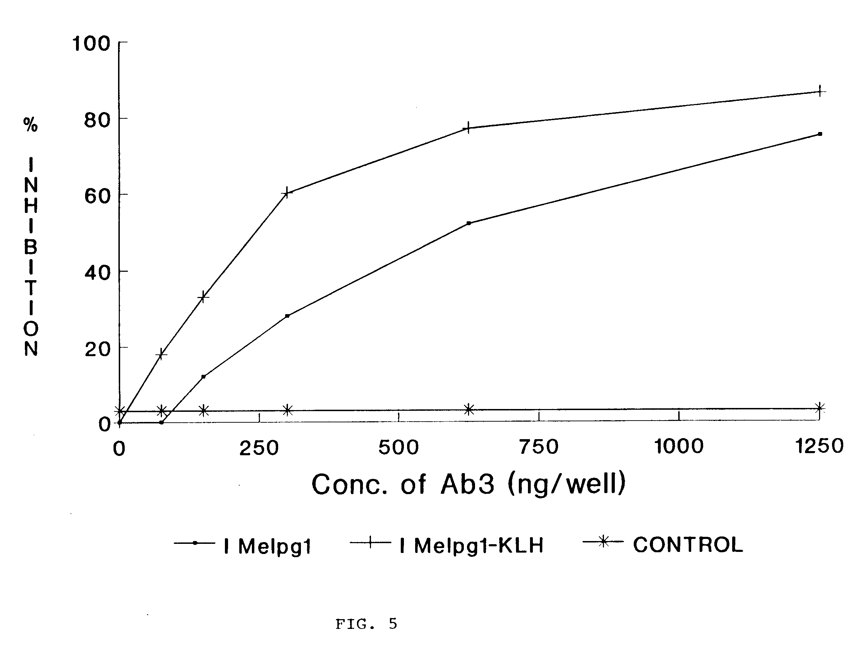

Figure 5 depicts inhibition of binding of Ab1 125I-225.28 to tumor cell line Colo 38 by rabbit Ab3s. Sera collected from rabbits immunized with IMelpg1, IMelpg1-KLH or ICO1 were affinity purified on an IMelpg1 column 7 days after second and fourth boost were tested for their ability to inhibit the binding of Ab1 $^{125}$I-225.28 to Colo 38 cells by a radioimmunoassay as described in the Materials and Methods section of Part II. The percentages of inhibition were computed and representative results obtained with two rabbits are shown.

The following terms, as used in this disclosure and claims, are defined as follows:

passive immunotherapy: passive transfer of antibody to a patient or animal.

active immunotherapy: the induction of antibody and/or T-cell responses in a patient or animal.

suppression: the inhibition of an immune response or the inhibition of the expression of an idiotypic response.

stimulation: the induction of an immune response or the induction of the expression of an idiotypic response.

tumor regression: an overall 30% or greater reduction of tumor.

I. Development and Characterization of the Anti-idiotypic Monoclonal Antibody (MF11-30) Directed to the Monoclonal Antibody 225.28 Directed Against HMW-MAA

The monoclonal antibody MF11-30 (ATCC, Rockville, MD deposit accession no. HB10133) is secreted by a hybridoma constructed with splenocytes from a BALB/c mouse immunized with anti-HMW-MAA monoclonal antibody 225.28 according to procedures similar to those described in Kusama, M., Kageshita, T., Perosa, F., and Ferrone, S. (1987), "Syngeneic Anti-idiotypic Antisera to Murine Antihuman High-Molecular Weight Melanoma-Associated Antigen Monoclonal Antibodies", Cancer Res. 47:4312-4317 (1987) for the development of syngeneic anti-idiotypic anti-sera to murine anti-HMW-MAA monoclonal antibodies. Kusama *et al* mentions antibody MF11-30 (For a description of antibody 225.28 see Wilson, B.S., International J. of Cancer 28:293-300 (1981). Antibody 225.28 available from ATCC, Rockville, MD as deposit accession no. HB10141). Briefly, purified monoclonal antibody 225.28 (200 $\mu$g) which had been coupled to keyhole limpet hemocyanin (sometimes referred to herein as "KLH") by glutaraldehyde fixation was injected intraperitoneally into BALB/c mice for use as immunogen. Complete and incomplete Freund's adjuvant were used as adjuvants to prime and boost the mice on day 0 and day 7, respectively. On day 14, the mice were boosted with monoclonal antibody 225.28 in phosphate buffered saline at pH 7.2. Three days later, the mice were sacrificed and the spleens were individually removed.

Hybridization of splenocytes with murine myeloma cells (P3-X63-Ag8.653) and subcloning were performed according to standard procedures known in the art (Kohler, G., Milstein, C., "Continuous cultures of fused cells secreting antibody of predefined specificity," Nature, 1975, 256:495-497) (hereby incorporated by reference). This fusion generated 293 different hybridomas. Screening of the supernatants with monoclonal antibody 225.28 in serological assays showed that 16 colonies reacted in an F(ab')$_2$ binding assay, four colonies reacted in a sandwich assay, and four inhibited the binding of monoclonal antibody 225.28 to the original antigen bearing melanoma cells (HMW-MAA), indicating that these latter antibody producing colonies produced an antibody directed to the paratope of the original antibody. The monoclonal antibody MF11-30 produced strong reactivity in all three assays, as shown in Figure 1 of the drawings and in Table 1. FIG. 1 shows inhibition by anti-idiotypic monoclonal antibody MF11-30 of the binding of $^{125}$I-labeled anti HMW-MAA monoclonal antibody 225.28 to cultured melanoma cells Colo 38.

The specificity of the MF 11-30 anti-id antibody as demonstrated in FIG. 1 was performed as described below. $^{125}$I-anti HMW-MAA monoclonal antibody 225.28 (FIG. 1 GRAPH A) and as a control, $^{125}$I-anti HMW-MAA monoclonal antibody TP41.2 (FIG. 1 GRAPH B) (1x10$^5$ cpm) were incubated for 2 hours at 4°C with anti-idiotypic monoclonal antibody MF11-30 (0-0) and with a control anti-idiotypic monoclonal antibody TK6-74 (.-.) specific for anti-HMW-MAA monoclonal antibody TP41.2. (The anti-idiotypic monoclonal antibody TK6-74 is secreted by a hybridoma constructed with splenocytes from a BALB/c mouse immunized with the anti HMW-MAA monoclonal antibody TP41.2). The mixtures were then added to cultured Colo 38 melanoma cells (2x10$^5$/well). At the end of a 1 hour incubation at 4°C, target cells were washed 5 times with phosphate-buffered saline and the bound radioactivity was measured in a gamma counter.

The results of serological assays testing the biological activity and reactivity of anti-idiotypic monoclonal antibody MF11-30 are set forth in the following Table 1. The anti-idiotypic monoclonal antibody TK6-74 was selected with the anti HMW-MAA monoclonal antibody TP41.2. The procedures for the binding assay used for these tests are described in Cancer Res., 47:4312-4317 (1987) The procedures for the sandwich assay used for these tests is described in Tsujisaki, M., Kusama, M., Sakaguchi, K., Perosa, F., and Ferrone, S., "A sandwich assay to detect and characterize syngeneic anti-idiotypic antibodies to murine anti-HLA and tumor associated antigen monoclonal antibodies," J. Immunol. Methods, 95:47-55 (1986).

Table 1

| Reactivity of anti-idiotypic monoclonal antibody MF11-30 in serological assays with anti HMW-MAA monoclonal antibodies | | | |
|---|---|---|---|
| Anti-idiotypic MoAb | | 225.28 | |
| | $F(ab^1)^2$ binding assay (in cpm) | Sandwich assay | Inhibition of binding to melanoma cells (by % inhibition) |
| MF11-30 | 13000 | 13900 | 92.6 |
| TKG-74 | 328 | 200 | < 5 |
| Anti-idiotypic MoAb | | TP41.2 | |
| | $F(ab^1)^2$ binding assay (in cpm) | Sandwich assay | Inhibition of binding to melanoma cells (by % inhibition) |
| MF11-30 | 600 | 128 | 11.0 |
| TKG-74 | 15000 | 9053 | 91.0 |

Testing of the monoclonal antibody MF11-30 with a panel of monoclonal antibodies to distinct antigenic determinants of HMW-MAA failed to reveal any reactivity of significance except to 225.28, thus indicating that the binding site on the MF11-30 antibody was directed to an idiotope of the 225.28 antibody not shared by other antibodies directed to the same antigen. Testing of the MF11-30 antibody against distinct melanoma associated antigens and to monomorphic and polymorphic determinants of HLA Class I and Class II also detected reactivity only with the monoclonal antibody 225.28.

These analyses were performed as follows. Polyvinylchloride microtiter plates (Dynatech Laboratories, Alexandria, VA) were coated with the test monoclonal antibody by an overnight incubation of 50 μl/well of a monoclonal antibody solution 100 μg/ml, 0.1 M bicarbonate buffer, pH 9.5). Following three washings with phosphate-buffered saline containing 0.05% Tween® 20 (PBS-T20), coating of microtiter plates with monoclonal antibodies was monitored by testing with $^{125}$I-labeled anti-mouse Ig xenoantibodies. Monoclonal antibody coated plates were incubated with $^{125}$I-monoclonal antibody MF11-30 ($2x10^5$ cpm/well) for 16 hours at 4°C. After 5 washings with PBS-T20 bound radioactivity was counted in a gamma counter. Specific binding is calculated by subtracting cpm in control wells from cpm in experimental wells. The results are set forth in Table II.

## Table II

Reactivity with anti MAA and anti HLA monoclonal
antibodies of the anti-idiotypic monoclonal antibody
MF11-30 elicited with the anti HMW-MAA monoclonal
antibody 225.28[1]

| MoAb | Ig | Specificity | $^{125}$I-MoAb MF11-30 in cpm x $10^{-3}$ |
|---|---|---|---|
| 149.53 | G1 | HMW-MAA | .6 |
| 225.28 | G2a | HMW-MAA | 19.9 |
| 763.74 | G1 | HMW-MAA | .7 |
| 653.25 | G1 | HMW-MAA | .7 |
| 657.5 | G1 | HMW-MAA | .8 |
| 902.51 | G1 | HMW-MAA | .7 |
| TP41.2 | G1 | HMW-MAA | .2 |
| TP61.5 | G1 | HMW-MAA | .8 |
| CL203 | G1 | 96Kd-MAA | .6 |
| 376.94 | G2a | 100Kd-MAA | .6 |
| 345.134 | G2a | 115Kd-MAA | .4 |
| CR11-351 | G1 | HLA-A2,A28 | .7 |

8

| | | | |
|---|---|---|---|
| KS1 | G1 | HLA-A2,A28 | .8 |
| KS2 | G1 | HLA-A2,A28 | .6 |
| KS3 | G2b | HLA-B7,B27,Bw42, Bw54,Bw55,Bw67,Bw73 | .9 |
| KS4 | G2b | HLA-B7,B27,Bw42, Bw54,Bw55,Bw67,Bw73 | .5 |
| Q6/64 | G2a | HLA-B | .5 |
| CR10-131 | G2b | HLA-Class I | .6 |
| CR10-214 | G1 | HLA-Class I | .7 |
| CR10-215 | G1 | HLA-Class I | .7 |
| CR11-115 | G2b | HLA-Class I | .7 |
| CR1 | G1 | HLA-Class I | .8 |
| Q1/28 | G2a | HLA-Class I | .7 |
| 6/31 | G2a | HLA-Class I | .8 |
| W6/32 | G2a | HLA-Class I | .3 |
| NAMB-1 | G1 | $\beta_2$-microgloblin | .7 |
| B7/21 | G2a | HLA-DPw1 | .6 |
| KS5 | M | HLA-DQw1 | .5 |
| KS11 | M | HLA-DQw1 | .3 |
| KS15 | G1 | HLA-DQw1 | .6 |
| H-1-940-1 | G2a | HLA-DQw3 | .4 |
| H-6-237-1 | G1 | HLA-DQw3 | .4 |
| H-7-34-1 | G2a | HLA-DQw3 | .4 |
| H-7-354-1 | G2a | HLA-DQw3 | .7 |
| H-9-61-1 | G2a | HLA-DQw3 | .6 |
| KS13 | G2b | HLA-DQw3 | .8 |
| KS6 | G2b | HLA-DQw1, DQw3 | .5 |
| KS10 | G2b | HLA-DQw1, DQw3 | .5 |
| KS14 | G2b | HLA-DQw1, DQw3 | .6 |
| AC1.59 | M | HLA-DR1,4, w6,w8,w9 | .3 |
| CL413 | G2a | HLA-DR | .4 |
| KS8 | G2b | HLA-DR | .4 |
| KS9 | G1 | HLA-DR | .4 |

EP 0 380 607 B1

| | | | |
|---|---|---|---|
| KS12 | G2b | HLA-DR | .8 |
| Q2/70 | G1 | HLA-DR | .6 |
| CR11-462 | G1 | HLA-DP,DR | .6 |
| Q2/80 | G2a | HLA-DP,DR | .7 |
| 127 | G2a | HLA-DP,DR | .7 |
| 417 | G1 | HLA-DP,DR | .7 |
| 420 | G1 | HLA-DP,DR | .7 |
| 441 | G1 | HLA-DP,DR | .7 |
| Q5/13 | G2a | HLA-DP,DQ,DR | .8 |

Preliminary results by Dr. R.A. Miller (IDEC Pharmaceuticals Corporation, Mountain View, California) indicated that the serum from a rat immunized with monoclonal antibody MF11-30 displayed high reactivity with cultured human melanoma cells in serological assays relative to preimmunization serum. Preliminary results by Dr. S. Raychaudhuri (IDEC Pharmaceuticals Corporation, La Jolla, California) also indicated that MF11-30 in a syngeneic strain of mice can induce 225.28 idiotype-bearing antibodies that bind to a cultured human melanoma cell line. Thus, the present results indicate that the monoclonal antibody MF11-30 can induce the formation of antibodies reacting with human melanoma cells. Preliminary results have indicated that administration of the MF 11-30 antibody induces reduction of tumor burden in some patients with malignant melanoma.

II. Development and Characterization of the Anti-idiotypic Monoclonal Antibody (IMelpg1) Directed to the Monoclonal Antibody 225.28 Directed Against HMW-MAA

We have discovered and highly purified a second anti-idiotype monoclonal antibody, IMelpg1 (deposited with the ATCC, Rockville, MD, under accession no. HB10134), which, in our opinion, based on our evidence to date, is highly pure and superior to MF11-30 in inducing an anti-tumor response.

The anti-idiotype antibody IMelpg1 is produced by a hybridoma constructed from a fusion of splenocytes from a BALB/c mouse immunized with monoclonal antibody 225.28 and murine myeloma cell lines P3-X63-Ag8.653 (Kageshita, T., et al., Pigment Cell Res. 1:185 (1988)).

IMelpg1 was prepared by treating the MF11-30 cell line with BM Cycline (Boheringer-Mannheim) according to the instruction supplied by the manufacturer. At the end of the treatment, a mycoplasma test was conducted, using a gene probe kit (Gen-Probe, San Diego, CA), and the cell line was found to be free of any detectable mycoplasma contamination. To purify the parental cell line from any residual mycoplasma, it subsequently was injected into mice intraperitoneally. After it was grown in ascites, the cells were passed into mice a second time in order to insure that the MF11-30 cell line was totally free of mycoplasma.

An MF11-30 hybridoma grown in ascites from the second group of mice was tapped and grown in vitro in tissue culture media without any antibiotics. After four weeks of culture, cell culture supernatant was sent to Coriel for mycoplasma testing. The result of the Coriel mycoplasma test was negative.

Mycoplasma freed MF11-30 was subcloned under limiting dilution at 0.3 cell/well (30 cell/96 well plate) using RPMI containing 15% total calf serum, glutamine and gentamycin. After two weeks, wells which showed growth were harvested and their culture supernatants were tested for their ability to bind to 225.28. Supernatants also were tested for their ability to inhibit the binding of $^{125}$I225.28 to a melanoma cell line Colo 38. A total of 50-60 wells containing positive growth were tested and 15-20 wells were found to be positive for the above assays. One of the positive subclones was IMelpg1, was expanded and used for future analysis.

The specificity and the biological role of the Ab3 response induced by IMelpg1 has been tested. Specifically, we have (i) determined in different species whether IMelpg1 induces a melanoma-specific humoral immune response (Ab3); (ii) determined whether IMelpg1-induced Ab3s are biochemically similar to 225.28 as determined by the radioimmunoprecipitation method; and (iii) examined different formulations of IMelpg1 to determine optimal conditions for Ab3 induction.

The data presented herein indicates that in an allogeneic system in mice, conjugation of Ab2 to a carrier was required for induction of antibodies recognizing antigens on human melanoma cells. However, in

10

a xenogeneic system (rabbits), both unconjugated and conjugated Ab2 induced anti-tumor antibodies. Conjugated Ab2 was more efficacious, since only two immunizations were needed compared to four vaccinations with Ab2 alone. Further dissection of the Ab3 response indicates that a portion of the idiotype response is tumor-specific, as revealed by the ability of the Ab3s to inhibit the binding of a monoclonal anti-melanoma antibody to cells carrying melanoma antigen. The fact that Ab2 and Ab3 immunoprecipitate identical molecular species from melanoma cells reinforces the anti-tumor specificity of the Ab2-induced humoral response. In addition, the anti-tumor antibodies induced by Ab2 are not hemocyanin (carrier) cross-reactive. Collectively, the data presented hereafter indicate the prerequisites for successful idiotype immunotherapy and demonstrate the potential use of IMelpg1 as an active immunotherapeutic agent in human melanomas.

## A. Materials and Methods

Cell lines. The human melanoma cell line Colo 38 was grown in RPMI 1640 medium supplemented with 10% calf serum, 1% glutamine and 10 $\mu$g/ml of gentamycin sulfate.

Monoclonal antibodies. Monoclonal antibody 225.28 ($\Gamma$2a,K) was purified on a protein A-Sepharose® 4B column. The monoclonal antibody IMelpg1 was purified on a 225-Sepharose affinity column. The immunoclinical antibody ICO1 ($\Gamma$l,K) was used as an isotype-matched control for IMelpg1.

Conjugation of anti-Id IMelpgl to KLH. The coupling was done according to the method described in Raychaudhuri, S. et al., J. Immunol. 137:1743 (1987). Briefly, 4 ml of antibody stock solution (1 mg/ml) was mixed with KLH (1 mg/ml) in PBS. Freshly diluted glutaraldehyde solution (1%) was added to the antibody and KLH mixture to make a final concentration of 0.05%. The mixture was incubated for 1 hour at room temperature and dialyzed overnight against PBS.

Immunization of mice. Eight week old A/J mice (three in each group) were immunized with 50 $\mu$g of IMelpg1 unconjugated or conjugated with hemocyanin (KLH) emulsified with SAF-M adjuvant containing 50 $\mu$g of threonyl MDP per mouse (Allison, A.C., et al., J. Immunol. Methods 95:157 (1986)). Injections were done at footpads, tail-base and also subcutaneously at multiple sites. Immunizations were repeated twice with the same concentration of antigen every 2 weeks subcutaneously. As a control, the same numbers of mice were immunized with 50 $\mu$g conjugated or unconjugated ICO1, an unrelated isotype-matched antibody, or 50 $\mu$g KLH alone. Mice were bled 7 and 14 days after each immunization.

Immunization of rabbits. Adult NZW rabbits (two in each group) were immunized intradermally at different sites with 500 $\mu$g of IMelpg1 unconjugated or conjugated with KLH in SAF-M adjuvant containing 250 $\mu$g of threonyl MDP per rabbit. Rabbits were boosted with the same antigen concentration every two weeks and bled 7 and 14 days after each immunization. Each rabbit was immunized a maximum of four times. Control rabbits were immunized with 500 $\mu$g ICO1 or KLH alone.

Analysis of Idiotypic (Ab3) response. The idiotypic response induced by IMelpg1 was analyzed by a radioimmunoassay (RIA) as described in Raychaudhuri, S. et al., J. Immunol. 137:1743 (1987). Briefly, 100 ng of IMelpg1-F(ab)$^2$ fragment was coated onto microtiter plates for 2 hours at room temperature. The plates were washed with PBS and the incubated with 1% BSA in PBS for 1 hour. Serially diluted sera from different experimental and control groups were incubated on the wells in the presence of 20,000 cpm of $^{125}$I-225.28 for 18 hours. The plates were thoroughly washed and the radioactivity in each well was counted in a gamma counter. The results are expressed as percentages of inhibition of $^{125}$I-225.28 binding to IMelpg1-F(ab)$^2$ fragment.

Analysis of Abl' response in sera. To determine the melanoma-specific antibody response, rabbit sera were first adsorbed onto unrelated 3T3 cells ($10^6$) for 3 hr at 4°C to remove non-specific reactivities. Unabsorbed and 3T3-absorbed sera were then serially diluted in 1% BSA and tested by an EIA for their ability to bind to melanoma cell lines (Colo38 and M21) which express the melanoma antigen. Cells were cultivated on microtiter plates and fixed with 0.05% glutaraldehyde in PBS for 15 minutes. Thereafter, plates were blocked with 1% BSA containing 0.1 M glycine for 40 minutes. Plates were incubated with different dilutions of sera for 2 hours, washed with PBS and then incubated with goat anti-mouse IgG conjugated to peroxidase for 1 hour. The plates were extensively washed, the peroxidase substrate ABTS was added and the optical density was measured at 405 nanometers.

Affinity purification of Ab3. To delineate the fraction of Ab3 antibodies capable of binding to melanoma antigen (Ab1' antibodies), the sera were subjected to affinity purification. Sera were first absorbed by passage through a normal mouse Ig-Sepharose® 4B column to eliminate the anti-isotypic antibodies. The absorbed sample was then applied onto an IMelpg1-Sepharose® 4B affinity column. The bound antibodies were eluted with 0.1 M acetic acid and neutralized immediately. These anti-anti-idiotype antibodies were examined in detail for their ability to bind to cells carrying melanoma antigens.

Analysis of Abl' response in affinity purified antibodies. The affinity purified Ab3s were analyzed for their anti-melanoma activity by an RIA to test their capacity to bind to melanoma cells. Different concentrations of purified antibodies were incubated for 2 hours on melanoma cells which were grown and fixed on microtiter plates as described above.

The specificity of the anti-melanoma activity of the purified Ab3s was further demonstrated by an inhibition assay, in which the ability of the affinity purified Ab3s to inhibit the binding of $^{125I}$-225 to Colo38 cells was determined. A fixed concentration of $^{125}$I-225 (20,000 cpm) was mixed with different concentrations of purified Ab3, then added to Colo38 cells fixed on microtiter plates. After overnight incubation, the plates were washed and the retained radioactivity was counted in a gamma counter. The results are expressed as percentages of inhibition. As a control, the capacity of the sera to inhibit the binding of $^{125}$I-MEM136 to Colo38 cells was similarly assessed. MEM136 is a monoclonal anti-MPG antibody which binds to the HMW-MAA at a site different from 225.28; i.e., MEM136 and 225.28 do not cross-inhibit each other to bind to melanoma cell lines.

Examination of the cross-reactivity of anti-KLH antibodies. To examine whether the anti-tumor antibodies induced by anti-idiotype conjugated to KLH are KLH-cross-reactive, the following experiments were conducted. Sera collected from rabbits immunized with IMelpg1-KLH were separately adsorbed to KLH-Sepharose® 4B for 1 hour in Eppendorf tubes with gentle mixing. The tubes were centrifuged in an Eppendorf centrifuge for 2 minutes to separate the anti-KLH antibodies. The KLH-Sepharose®-depleted antibodies were then assessed for their ability to bind to Colo38 cells by an RIA as described earlier.

As a corollary experiment, the affinity purified Ab3s were also tested for binding to KLH by an RIA. 100 ng of KLH in PBS per well was coated onto microtiter plates for 18 hours at 4°C. The plates were washed and then incubated with 1% BSA for 1 hour. The affinity purified Ab3s were incubated on the plates for 2 hours at room temperature. After washing the plates, 20,000 cpm of iodinated goat anti-rabbit Igs were added and incubated overnight at 4°C. The plates were washed and the radioactivity counted.

Immunoprecipitation of HMW-MAA. Colo 38 cells (1.5 x 10$^7$) were biosynthetically labelled for 3 hours with 200 μCi of $^{35}$S-methionine in 1 ml of methionine-free RPMI medium (See Nepom, G.T., et al., Proc. Natl. Acad. Sci. U.S.A. 81:2864 (1984). Cells were centrifuged and the pellet was solubilized in RIPA-lysis buffer (10 mM Tris, 150 mM NaCl, 1% deoxycholate, 1% Triton® X-100, 0.1% SDS, 1 mM aprotinin, 10 mM PMSF, 5 mM N-ethylmaleimide) for 1 hour on ice. The lysate was cleared by centrifuging at 110,000 g for 45 minutes. Lysate corresponding to 5 x 10$^7$ cpm was added to a pre-prepared immunoadsorbent containing 100 μg of Ab2 affinity purified Ab3s adsorbed to 10% protein A-Sepharose® in a 100 μl volume. As a control, normal mouse IgG eluted antibodies were added to protein A-Sepharose. After incubating for 18 hours at 4°C with gentle mixing, the immunoadsorbants were washed three times with PBS containing 1% ovalbumin and 0.5% Tween®-20. The antigens were released from protein A-Sepharose® by boiling for 2 minutes in sample buffer and applied onto SDS-PAGE (6%). The gel was dried and exposed to film with intensifying screens at -70°C for 48 hours.

B. Results

Ab3 induction in mice. In our preclinical murine studies we maximized the Ab2-induced immune response by injecting the BALB/c-derived IMelpg1 into an allogeneic strain of mice. A/J mice were used for this study. As will be shown below, our findings indicate that IMelpg1 is capable of inducing an anti-melanoma response in a xenogenic setting (i.e., melanoma patients).

Mice immunized with unconjugated IMelpg1 did not produce any Ab3 response even after four immunizations (data not shown). Mice that received KLH-conjugated IMelpg1 produced an idiotype-bearing anti-tumor IgG response. Table III shows the Ab3 response induced in each mouse by IMelpg1-KLH. No significant anti-tumor response was observed in control mice immunized with KLH alone. As shown in Table III (right column), 20-30% of Colo38-binding antibodies could be inhibited by 225.28.

Table III

| Induction of anti-tumor antibodies by IMelpg1 anti-idiotype | | |
|---|---|---|
| | Titer of antibody ($\mu$g/ml) | |
| | Anti-Colo38[a] | 225 idiotype[b] |
| KLH | 1:100 (1.6) | - |
| | 1:100 (1.6) | - |
| | 1:200 (3.2) | - |
| IMelpg1-KLH | 1:1600 (25.6) | 20-30% |
| | 1:800 (12.8) | 20-30% |
| | 1:1600 (25.6) | 20-30% |

[a] Different dilutions of experimental (IMelpg1-KLH-immunized) and control (KLH-immunized) sera (bled 7 and 14 days after the second immunization) were added to plates coated with the Colo38 cell line. Thereafter plates were developed with peroxidase-labeled goat anti-mouse IgG. Data from three individual mice are shown. Different concentrations of 225 were used as antibody control.

[b] 225 idiotype (anti-chondroitin sulfate) analysis was done in a competitive inhibition assay where 20,000 cpm of 225 was added to each well containing Colo38 (melanoma cell line) and different dilutions of experimental or control sera. Plates were incubated overnight.

Analysis of the Ab3 response in rabbits. To maximize the immunogenicity of mouse Ab2s even further, we immunized rabbits with IMelpg1 and investigated whether, in the xenogeneic situation, carrier conjugation is needed or useful for an Ab2 to induce an anti-tumor humoral response. Rabbits were immunized with IMelpg1 alone or with IMelpg1-KLH in SAF-M. Rabbits were immunized with KLH alone as a control for the KLH-conjugated IMelpg1 immunized group, and with ICO1 as a control for the unconjugated IMelpg1 immunized group. Rabbits immunized twice intradermally with unconjugated IMelpg1 in SAF-1 adjuvant did not produce anti-melanoma antibodies, although an idiotype response was observed. However, a low level anti-tumor humoral response was observed after four immunizations. The data are presented in Figure 2, and demonstrate that, compared to the control, IMelpg1 induced a significant anti-tumor humoral response in rabbits after repeated immunizations.

Rabbits immunized with KLH conjugated to IMelpg1 also produced a significant anti-tumor humoral response. The data from a representative experiment are presented in Figure 3a and b. Figure 3 shows the tumor binding of different dilutions of serum from rabbits immunized with KLH-IMelpg1 before and after absorption with unrelated mouse tumor cells. These data demonstrate that IMelpg1-KLH immunization induced an Ab3 response which preferentially bound to the melanoma Colo38 cell line. The control sera, i.e., sera from rabbits immunized with KLH alone, did not produce any tumor binding antibodies.

Affinity purification of Ab3s from sera. To conclusively determine that it is the Ab3 that binds to the melanoma cell line Colo38, we affinity purified Ab3s on an IMelpg1-Sepharose® column. IMelpg1 immune sera was first passed through a normal rabbit IgG column to absorb anti-isotypic activity. The material not bound by normal mouse Ig was passed through an IMelpg1-Sepharose® column. The bound material was eluted and assayed for anti-melanoma activity on the melanoma cell line (Fig. 4a). The specificity of Ab3 binding was determined in an inhibition assay, where binding of $^{125}$I-225 to the Colo38 cell line was determined in the presence of different concentrations of Ab3s. The results are shown in Figure 5, and demonstrate that $^{125}$I-225 binding to tumor could be inhibited by purified Ab3s.

KLH cross-reactivity of Ab2-induced anti-tumor antibodies. To determine whether anti-tumor antibodies are KLH cross-reactive, two sets of experiments were done. In the first set, KLH-binding antibodies were removed from the sera sample. Individual sera collected from IMelpg1-KLH-immunized rabbits were incubated separately with KLH-Sepharose 4B in Eppendorf tubes for 1 hour with intermittent mixing. Thereafter tubes were centrifuged and the unbound material was tested for its ability to bind to Colo38 and KLH. After removal of KLH-binding antibodies, the rabbit sera were still able to bind to the Colo38 cell line, but not to KLH (data not shown). In the second series of experiments, the IMelpg1 affinity purified Ab3s were tested for their ability to bind to KLH. The data are presented in Figure 4b, and demonstrate that Ab3s purified on an IMelpg1 column are melanoma-specific and do not significantly bind to KLH.

Immunoprecipitation of HMW-MAA by Ab1 and Ab3. Radioimmuno-precipitation was performed to determine whether the Ab1s and Ab3s recognize the same molecular species. Immunoprecipitation of HMW-MAA of Colo38 cells by different antibodies was carried out as described in the Materials and Methods section, supra. 225.28 was used as a positive control. CC92 which is melanoma-unrelated was used as a negative control. The anti-id affinity purified Ab3s from two different rabbits immunized four times with anti-id IMelpg1 were used for immunoprecipitation. It appeared that Ab1 and Ab3 recognized the same molecular species, which was approximately 250 K in size. Furthermore, immunoprecipitation by Ab3 was shown to be specific, as it was appreciably depleted by pretreatment of the melanoma lysate with 225.28.

Discussion

Anti-ids can be used as immunoregulators to either stimulate or suppress an antigen-specific response (See Cosenza, H. et al., Science 176:1027 (1972); Thomas, W.R. et al., J. Immunol. 130:2079 (1983). Here we have studied an anti-tumor immune response induced by Ab2. In particular, these studies describe the specificity and the biological role of an Ab3 response induced by an Ab2. Our data can be summarized as follows: (i) for the efficient induction of an anti-tumor humoral response in mice, murine Ab2 needs to be conjugated to a carrier; (ii) unconjugated Ab2 can induce an anti-tumor humoral response in rabbits after repeated immunizations, while carrier-conjugated Ab2 induces an anti-tumor humoral response after only two immunizations; (iii) a portion of the total idiotype response is tumor-specific (see Fig. 3a; 225.28 and Ab3 binding to the melanoma cell line); (iv) the binding of a monoclonal anti-melanoma antibody to a melanoma cell line can be inhibited by Ab3s; (v) the anti-tumor antibodies induced by Ab2 are not hemocyanin cross-reactive; and (vi) Ab1 and Ab3 precipitate similar molecular mass species from a melanoma cell line.

Neither the Ab1 nor the Ab3 mediate complementdependent cytotoxicity or antibody-dependent cellular cytotoxicity (data not shown). This lack of killing activity is most likely due to the location of the antigen in the extracellular matrix (ECM). Therefore, if there is any positive association between Ab3 induction and melanoma growth, it cannot be due to Ab3-mediated killing of tumor cells. On the other hand, Ab3 may mediate an even more profound function which interferes with the biology of tumor cell attachment to the basal membrane, thus minimizing the metastatic potential of melanoma cells.

It is known that tumor cells traverse ECM during the process of invasion and metastasis. The ECM which is most relevant to metastasis is the basal lamina or basement membrane. Basement membranes are composed mainly of collagen type IV meshed laminin, entactin, fibronectin and proteoglycans. A majority of experimental evidence suggests a close correlation between extracellular membrane degradation and tumor cell invasion and metastasis. Therefore, Ab3 attachment to ECM may interfere with the biology of tumor growth and metastasis by inhibiting the binding of tumor cells to ECM.

Taken together, our data demonstrate the utility of IMelpg1 as an active melanoma immunotherapeutic agent. IMelpg1 also may be useful in inducing protective immunity.

III. Use of the Anti-idiotype Antibodies in Diagnosis, Monitoring and Immunotherapy

The anti-idiotypic antibodies described herein may be used in disease diagnosis and monitoring and in active or passive immunotherapy according to the following method. (Although the following discussion will describe the use of MF11-30 in disease diagnosis and monitoring and in active or passive immunotherapy, those skilled in the art will recognize that the methods apply, as well, to IMelpg1). In disease diagnosis and monitoring MF11-30 may be incorporated into in vitro assays (such as ELISA or RIA) in order to measure the levels of serum idiotypes reactive with MF11-30 before, during and after immunotherapy; for example, in measuring the booster effect of multiple administrations of MF11-30 in active immunotherapy.

In active immunotherapy, MF11-30 may be administered in order to heighten the immune response to stimulate beneficial components of the immune system; for example, in treatment of malignant melanoma it is desirable to elicit humoral and cellular responses directed against the melanoma associated antigen.

When used in active immunotherapy, the anti-idiotype antibodies may be administered in an adjuvant formulation meaning, adjuvants, carriers, vehicles and conjugation techniques known or developed by those skilled in the art. (This is preferred for IMelpg1). Such formulations may incorporate Syntex Adjuvant Formulation-1 (SAF-1), muramyl dipeptide derivatives, lipopolysaccharides, pluronic polymers, Bacillus Calmette-Guérin (BCG), liposomes, mineral oil emulsions, alum adjuvants, such as aluminum hydroxide and aluminum phosphate, saponins such as Quil A, immune-stimulating complexes (ISCOMS), lipid A, keyhole limpet hemocyanin (KLH), hepatitis core antigen, tetanus toxoid, water-in-oil emulsions, glutaraldehyde crosslinking and other conjugation procedures to conjugate the anti-idiotype antibodies to adjuvants or carriers. Examples of adjuvant formulations incorporating these materials and processes have been described in Steinberger, A. et. al, Experimental Parasitology 58:223-229 (1984); Morein, B., Nature 332: 287-288 (1988); Allison, A.C., J. Immunol. Methods 95:157-168 (1986); Clarke, B.E., et. al, Nature 330:381-384 (1987); Raychaudhuri, S., et. al J. Immunol. 137:1743 (1986); Eskola, J. et. al New Eng. J. Med. 317:717-722 (1987); Allison, A.C. Bio/Technology 5:1041 (1987); Leonard J.E., et. al, Blood 65:1149-1157 (1985); Practical Guide for Use in Affinity Chromatography and Related Techniques (2d ed. 1983 by Reactiff IBF) pub. Societe Chemique Pointet-Girard, France.

When used in active immunotherapy, the antibodies are administered to the patient in a manner and dose which will induce tumor regression. They normally will be administered by subcutaneous or intramuscular (IM) injection. The proper dosage of Ab2 to be administered to a particular patient will depend on the patient's size and the status of their immune system function. Dosages may range from 500 $\mu$g to 5 mg. The likely preferred dosage range to be administered would be 100 $\mu$g to 5mg per injection site. Of course, the objective is to use the minimum dose shown to provide efficacious humoral and/or cellular antitumor responses. Multiple injection sites may be used.

With regard to the use of the antibodies in passive immunotherapy, there may be instances of disease (for example, auto-immune disease) in which HMW-MAA is a target of deleterious B-cell or T-cell immune responses that are attacking healthy tissues. Passive immunotherapy may be used in this circumstance of disease. When used in passive immunotherapy, the antibodies herein disclosed are not used in adjuvant formulations. The method of administration is intravenous injection and generally, larger doses of antibody are given (in the range of 50 mg to 5g). Other uses of these antibodies in passive immunotherapy will be apparent to those skilled in the art. Generally, passive immunotherapeutic uses of these antibodies will be appropriate whenever the HMW-MAA is the target of immune responses that one wishes to suppress.

**Claims**

1. The murine anti-idiotypic antibody IMelpg1 obtained from the hybridoma deposited with the ATCC under accession no. HB 10134 and antibodies competing with said antibody IMelpg1 to bind to the murine monoclonal antibody 225.28 obtained from the hybridoma deposited with the ATCC under accession no. HB 10141, said antibodies and said antibody IMelpg1 being free of mycoplasma contamination.

2. The use of an anti-idiotypic antibody of claim 1 for *in vitro* assays.

3. A formulation comprising an anti-idiotypic antibody of claim 1 and an adjuvant, adjuvant formulation, carrier or vehicle.

4. The formulation of claim 3 wherein the adjuvant is a muramyl dipeptide derivative, lipopolysaccharide, Bacillus Calmette-Guérin (BCG), alum adjuvant, aluminum hydroxide, aluminum phosphate, saponin, Quil A or lipid A.

5. The formulation of claim 3 wherein the adjuvant formulation is Syntex Adjuvant Formulation-1 (SAF-1) or an immune stimulating complex (ISCOM).

6. The formulation of claim 3 wherein the carrier is a keyhole limpet hemocyanin (KLH), hepatitis core antigen, or tetanus toxoid.

7. The formulation of claim 3 wherein the vehicle is a water-in-oil emulsion, mineral oil emulsion, liposome or poly(oxypropylene)poly(oxyethylene)copolymer surfactant.

8. A formulation of an anti-idiotypic antibody of claim 1 which has been conjugated to an adjuvant or carrier by a glutaraldehyde crosslinking or covalent conjugation procedure.

9. The anti-idiotype antibody secreting hybridoma deposited with the ATCC, Rockville, MD, under accession no. HB 10134.

10. An anti-idiotype antibody as claimed in claim 1 or obtained from a hybridoma as claimed in claim 9 for use as a medicament in a method of medical treatment.

11. Use of an anti-idiotype antibody as claimed in claim 1 or a hybridoma as claimed in claim 9 in the manufacture of a medicament for the treatment of a melanoma.

**Patentansprüche**

1. Muriner anti-idiotyper Antikörper IMelpg1, erhalten aus dem von der ATCC unter der Zugangsnummer HB 10134 hinterlegten Hybridom, und Antikörper, die mit dem Antikörper IMelpg1 um die Bindung an den murinen monoklonalen Antikörper 225.28, erhalten aus dem von der ATCC unter der Zugangsnummer HB 10141 hinterlegten Hybridom, kompettitieren, wobei die Antikörper und der Antikörper IMelpg1 keine Mycoplasma-Kontamination aufweisen.

2. Verwendung des anti-idiotypen Antikörpers nach Anspruch 1 für *in vitro*-Assays.

3. Formulierung, die einen anti-idiotypen Antikörper nach Anspruch 1 und ein Adjuvans, eine Adjuvans-Formulierung, einen Träger oder ein Vehikel umfaßt.

4. Formulierung nach Anspruch 3, worin das Adjuvans ein Muramyldipeptidderivat, Lipopolysaccharid, Bacillus Calmette-Guérin (BCG), Alaun-Adjuvans, Aluminiumhydroxid, Aluminiumphosphat, Saponin, Quil A oder Lipid A ist.

5. Formulierung nach Anspruch 3, worin die Adjuvansformulierung Syntex-Adjuvans-Formulierung-1 (SAF-1) oder ein immunstimulierender Komplex (ISCOM) ist.

6. Formulierung nach Anspruch 3, worin der Träger "Schlüsselloch-Napfschnecken"-Hämocyanin (KLH), Hepatitis-Kern-Antigen oder Tetanustoxoid ist.

7. Formulierung nach Anspruch 3, worin das Vehikel eine Wasser-in-Öl-Emulsion, eine Mineralölemulsion, ein Liposom oder Poly(oxypropylen)poly(oxyethylen)-Copolymer-Tensid ist.

8. Formulierung eines anti-idiotypen Antikörpers nach Anspruch 1, welcher an ein Adjuvans oder einen Träger mittels einer Glutaraldehydvernetzung oder einer kovalenten Verknüpfung gebunden worden ist.

9. Anti-idiotype Antikörper sekretierendes Hybridom, das von der ATCC, Rockville, MD, unter der Zugangsnummer HB 10134 hinterlegt ist.

10. Anti-idiotyper Antikörper nach Anspruch 1 oder erhalten aus einem Hybridom nach Anspruch 9 zur Anwendung als ein Medikament bei einem Verfahren zur medikamentösen Behandlung.

11. Verwendung eines anti-idiotypen Antikörpers nach Anspruch 1 oder eines Hybridoms nach Anspruch 9 bei der Herstellung eines Medikamentes zur Behandlung eines Melanoms.

**Revendications**

1. Anticorps anti-idiotypique murin IMelpg1 obtenu à partir de l'hybridome déposé à l'ATCC sous le no. HB 10134 et anticorps entrant en compétition avec ledit anticorps IMelpg1 pour la liaison avec l'anticorps monoclonal murin 225.28 obtenu à partir de l'hybridome déposé à l'ATCC sous le no. HB10141, lesdits anticorps et ledit anticorps IMelpg1 étant dépourvus de contamination par des mycoplasma.

2. Utilisation d'un anticorps anti-idiotypique de la revendication 1 pour des essais *in vitro*.

3. Formulation comprenant un anticorps anti-idiotypique de la revendication 1 et un adjuvant, un adjuvant de formulation, un support ou un véhicule.

4. Formulation de la revendication 3 dans laquelle l'adjuvant est un dérivé du muramyl dipeptide, un lipopolysaccharide, le bacille Calmette-Guérin (BCG), l'adjuvant alun, l'hydroxyde d'aluminium, le phosphate d'aluminium, une saponine, le Quil A ou le lipide A.

16

**5.** Formulation de la revendication 3 dans laquelle la formulation de l'adjuvant est l'Adjuvant Syntex Formulation-1 (SAF-1) ou un complexe immunostimulant (ISCOM).

**6.** Formulation de la revendication 3 dans laquelle le support est une hémocyanine de mollusque keyhole limpet (KLH), un antigène de core de l'hépatite, ou un toxoïde tétanique.

**7.** Formulation de la revendication 3 dans laquelle le véhicule est une émulsion eau-dans-huile, une émulsion d'huile minérale, un liposome ou un tensioactif copolymère poly(oxypropylène)poly-(oxyéthylène).

**8.** Formulation d'un anticorps anti-idiotypique de la revendication 1 qui a été conjugué à un adjuvant ou un support par un procédé de réticulation au glutaraldéhyde ou de conjugaison par covalence.

**9.** Hybridome secrétant un anticorps anti-idiotypique déposé à l'ATCC, Rockville, MD, sous le no. HB 10134.

**10.** Anticorps anti-idiotypique tel que revendiqué dans la revendication 1 ou obtenu à partir d'un hybridome tel que revendiqué dans la revendication 9 pour l'utilisation comme médicament dans une méthode de traitement médical.

**11.** Utilisation d'un anticorps anti-idiotypique tel que revendiqué dans la revendication 1 ou d'un hybridome tel que revendiqué dans la revendication 9 dans la fabrication d'un médicament pour le traitement d'un mélanome.

FIGURE 1

225.28

TP41.2

GRAPH A

GRAPH B

EP 0 380 607 B1

FIG. 2

FIG. 3

EP 0 380 607 B1

FIG. 4

FIG. 5

EP 0 380 607 B1